# EUROPEAN PATENT APPLICATION

(11) **EP 4 173 585 A1**
(43) Date of publication of application: **03.05.2023**
(21) Application number: 21218158.0
(22) Date of filing: 29.12.2021
(51) Int. Cl.: A61B 34/10

(54) **METHOD FOR IDENTIFYING A VASCULAR ACCESS SITE**

(30) Priority: 27.10.2021 US 202163272251 P
(71) Applicant: Koninklijke Philips N.V., 5656 AG Eindhoven (NL)
(72) Inventor: SINHA, Ayushi, Eindhoven (NL); FLEXMAN, Molly Lara, Eindhoven (NL); GUPTA, Atul, Eindhoven (NL); PANSE, Ashish Sattyavrat, Eindhoven (NL); SALEHI, Leili, Eindhoven (NL); TOPOREK, Grzegorz Andrzej, Eindhoven (NL); ERKAMP, Ramon Quido, Eindhoven (NL)
(74) Representative: Philips Intellectual Property & Standards

(57) **Abstract**

A computer-implemented method of identifying a vascular access site for inserting an interventional device in order to reach a target site in a vasculature, is provided. The method includes computing a success metric for multiple potential vascular access sites. The success metric represents an ease of navigating the interventional device from the vascular access site to the target site via the vasculature, and is computed based on image data. A vascular access site is identified based on the computed success metrics.

## Description

### TECHNICAL FIELD

The present disclosure relates to identifying a vascular access site for inserting an interventional device in order to reach a target site in a vasculature. A computer-implemented method, a computer program product, and a system, are disclosed.

### BACKGROUND

Interventional procedures in the vasculature can often be performed by choosing one of multiple vascular access sites to reach an intended target site. For example, percutaneous coronary interventions "PCI" procedures can often be performed with either radial access, or femoral access. The choice of the access site is typically made by a physician, and is based on multiple factors such as the ease of navigating an interventional device from the access site to the target site, and the expected outcome of performing a vascular intervention at the target site using the vascular access site.

By way of an example, a document by Jolly, S. et al., entitled "Radial versus femoral access for coronary angiography or intervention and the impact on major bleeding and ischemic events: A systematic review and meta-analysis of randomized trials," American Heart Journal 157(1): 132-140 (2009), reports a meta-analysis of 23 randomized trials in which PCI procedures with radial access had a 73% lower chance of bleeding, a 0.4 day shorter hospital stay, and a trend toward reduced occurrence of stroke, as compared to those with femoral access. However, this meta-analysis also showed that procedures with radial access showed a trend toward increased inability to cross the target lesion with a wire, balloon, or stent.

Multiple factors may therefore influence a physician's choice of a vascular access site, including the location of the target site, the tortuosity of the vasculature leading to the target site, the presence of obstructions such as stenoses or calcification in the vasculature that may hamper navigation to the target site. The choice of vascular access site may also impact the outcome of an interventional procedure performed at the target site, such as for example the procedure time, and the risk of complications. For example, if a sub-optimal access site is chosen, the target site may not be reachable and the procedure may have to be restarted from a new access site, lengthening the procedure time. Similarly, if the vasculature is heavily calcified or has eccentric plaque or thrombus, the navigation of interventional devices risks complications such as bleeding, vascular injury including dissection, extravasation and rupture, or even embolus.

Due to the multitude of factors that affect the choice of a vascular access site to perform an interventional procedure at a target site, a physician may make an intuitive decision as to which access site to use. Alternatively, the physician may search through electronic health record "EHR" data for a patient in order to make a more informed choice. However, this process is inefficient because extracting all the relevant information from various sources and mentally resolving the information into an optimal choice of vascular access site is complex and time-consuming. A sub-optimal selection of a vascular access site can lengthen the procedure time, or even harm the patient if stenoses, occlusions, heavy calcifications or even foreign bodies such as implanted devices are encountered.

Consequently, there remains room to improve the task of choosing a vascular access site in order to perform an interventional procedure.

### SUMMARY

According to one aspect of the present disclosure, a computer-implemented method of identifying a vascular access site for inserting an interventional device in order to reach a target site in a vasculature, is provided. The method includes:
receiving input indicative of the target site;
receiving image data representing at least a portion of the vasculature; and
for a plurality of potential vascular access sites:
   computing, based on the image data, a success metric representing an ease of navigating the interventional device from the vascular access site to the target site via the vasculature; and
   identifying the vascular access site based on the computed success metrics.

The result of these operations is to identify an optimal vascular access site for the target site. The optimal vascular access site may then be recommended to a physician. The optimal vascular access site is also provided in a consistent and efficient manner, obviating some of the existing challenges in determining an optimal access site.

Further aspects, features, and advantages of the present disclosure will become apparent from the following description of examples, which is made with reference to the accompanying drawings.

### BRIEF DESCRIPTION OF THE DRAWINGS

Fig. 1 is a flowchart illustrating an example of a method of identifying a vascular access site for inserting an interventional device in order to reach a target site in a vasculature, in accordance with some aspects of the present disclosure.
Fig. 2 is a schematic diagram illustrating an example of a system 200 for identifying a vascular access site for inserting an interventional device in order to reach a target site in a vasculature, in accordance with some aspects of the present disclosure.
Fig. 3 is a schematic diagram illustrating an example of an anatomical image including an identification of a location of a dominant feature affecting one or more of the success factors, in accordance with some aspects of the present disclosure.
Fig. 4 is a schematic diagram illustrating an example of a graphical user interface 160 for providing a ranking of potential vascular access sites, in accordance with some aspects of the present disclosure.
Fig. 5 is a schematic diagram illustrating a first example of a neural network 170 for use in identifying a vascular access site for inserting an interventional device in order to reach a target site 130 in a vasculature, in accordance with some aspects of the present disclosure.
Fig. 6 is a schematic diagram illustrating a second example of a neural network 170 for use in identifying a vascular access site for inserting an interventional device in order to reach a target site 130 in a vasculature, in accordance with some aspects of the present disclosure.
Fig. 7 is a flowchart illustrating an example of a method of training a neural network to predict a success metric and/or to identify a vascular access site, in accordance with some aspects of the present disclosure.

### DETAILED DESCRIPTION

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a computer implemented method, may be implemented in a system, in a corresponding manner.

In the following description, reference is made to computer-implemented methods that involve identifying a vascular access site for inserting an interventional device in order to reach a target site in a vasculature. Reference is made to an example of a PCI procedure in which femoral or radial access is used to reach the coronary arteries. However, it is to be appreciated that the disclosure is not limited to use in performing coronary interventional procedures, or to use with these example access sites. The target site, may therefore be anywhere in the vasculature.

A vascular access site is in general a superficial position on the body that provides access to an artery, or to a vein of the vasculature. Examples of vascular access sites include a femoral vascular access site, i.e. a superficial position on the body that provides access to the femoral artery, a radial vascular access site, i.e. a superficial position on the body that provides access to the radial artery, a brachial arterial access site, an axillary arterial access site, and a jugular access site. However, is to be appreciated that the disclosure is not limited to use with these examples of access sites, and that the access site may alternatively be at any suitable superficial position on the body that provides access to an artery, or to a vein of the vasculature.

Reference is made herein to a PCI procedure as an example of an interventional procedure that is performed at the target site. A PCI procedure is used to restore blood flow to a coronary artery, and typically involves the insertion of a balloon catheter from a femoral or radial access site, to a target site in the coronary arteries. The balloon is inflated inside a stent at the target site in order to open-up the artery. However, it is to be appreciated that the methods disclosed herein are not limited to this example PCI procedure, or to this example of an interventional device. Thus, the methods may be used with various types of interventional procedures, and as appropriate, with various types of interventional devices, such as, and without limitation: a guidewire, a catheter, a balloon catheter, an atherectomy device, an intravascular ultrasound "IVUS" imaging device, an Optical Coherence Tomography "OCT" imaging device, a blood pressure device and/or flow sensor device.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

As mentioned above, multiple factors may influence a physician's choice of a vascular access site to perform an interventional procedure at a target site. Consequently, a physician may make an intuitive decision as to which access site to use. Alternatively, the physician may search through electronic health record data for the patients in order to make a more informed choice. However, this process is inefficient because extracting all the relevant information from various sources and mentally resolving the information into an optimal choice of vascular access site is complex and time-consuming.

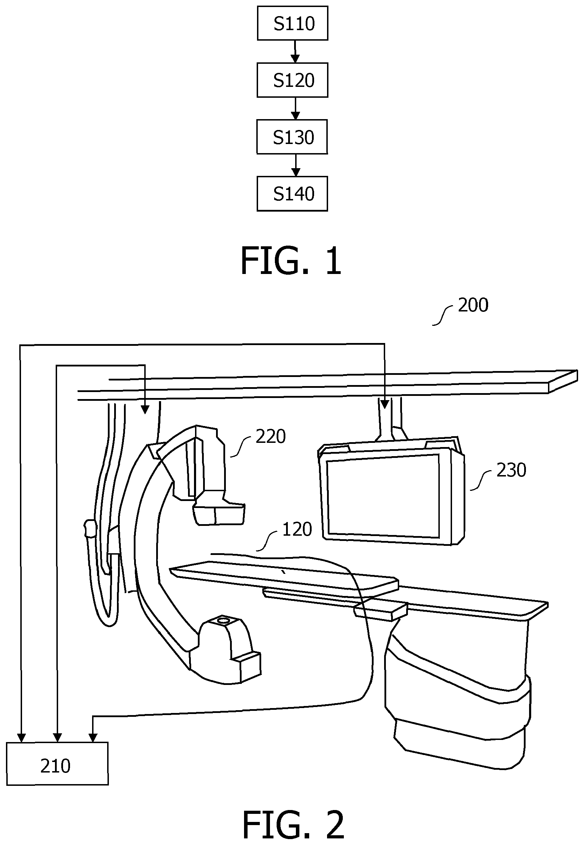

Fig. 1 is a flowchart illustrating an example of a method of identifying a vascular access site for inserting an interventional device in order to reach a target site in a vasculature, in accordance with some aspects of the present disclosure. With reference to Fig. 1, the method includes:
receiving S110 input indicative of the target site 130;
receiving S120 image data 140 representing at least a portion of the vasculature; and
for a plurality of potential vascular access sites 110_{1..n}:
   computing S130, based on the image data 140, a success metric 150 representing an ease of navigating the interventional device 120 from the vascular access site 110 to the target site 130 via the vasculature; and
   identifying S140 the vascular access site 110 based on the computed success metrics.

The result of these operations is to identify an optimal vascular access site for the target site. The optimal vascular access site may then be recommended to a physician. The optimal vascular access site is also provided in a consistent and efficient manner, obviating some of the existing challenges in determining an optimal access site.

The method described above may also be implemented by the system 200 illustrated in Fig. 2, which is a schematic diagram illustrating an example of a system 200 for identifying a vascular access site for inserting an interventional device in order to reach a target site in a vasculature, in accordance with some aspects of the present disclosure. Operations described in relation to the flowchart illustrated in Fig. 1, may also be performed by the system 200 illustrated in Fig. 2, and vice versa.

With reference to Fig. 1, in the operation S110, input indicative of the target site 130, is received. By way of an example, the target site may for example be a location in the coronary arteries in which it is intended to perform an interventional procedure, such as a PCI procedure. The input may be received by the one or more processors 210 illustrated in Fig. 1. The input may be received from a user input device (not illustrated in Fig. 1) such as a keyboard, a mouse, a touchscreen, a joystick, and so forth. The user may use the user input device to select the target site from a list of possible target sites using a drop-down menu, for example. By way of another example, the user may identify the target site by clicking via a mouse, or touching via a touchscreen, on a position in an image representing a portion of the anatomy. The input may be received via any form of data communication, including wired, optical, and wireless communication. By way of some examples, when wired or optical communication is used, the communication may take place via signals transmitted on an electrical or optical cable, and when wireless communication is used, the communication may for example be via RF or optical signals.

In the operation S120 image data 140 representing at least a portion of the vasculature, is received. In this respect, the image data 140 may include one or more types of image data, such as for example: intravascular ultrasound "IVUS" image data, optical coherence tomography "OCT" image data, computed tomography angiography "CTA" image data, magnetic resonance "MR" image data, MR angiography "MRA" image data, digital subtraction angiography "DSA" image data, and positron emission tomography "PET" image data. The image data 140 may be received by the one or more processors 210 illustrated in Fig. 2. The image data 140 may be received via any form of data communication, as described above in relation to the input indicative of the target site 130.

In the operation S130, a success metric 150 is computed for a plurality of potential vascular access sites 110_{1..n}, based on the image data 140. The potential vascular access sites may include sites such as a femoral access site, a radial access site, a brachial arterial access site, an axillary arterial access site, and a jugular access site, for example. The success metric represents an ease of navigating the interventional device 120 from the vascular access site 110 to the target site 130 via the vasculature.

By way of an example the interventional device 120 may be a balloon catheter. The success metric may be computed based on the value of one or more success factors that affect the ease of navigating the interventional device from the vascular access site 110 to the target site 130 via the vasculature. In this regard, the success factors may represent features such as:
a tortuosity of a portion of the vasculature between the vascular access site 110 and the target site 130;
a difficulty of passing a stenosis in the vasculature between the vascular access site 110 and the target site 130;
a difficulty of passing an implanted device in the vasculature between the vascular access site 110 and the target site 130;
a difficulty of passing a calcification in the vasculature between the vascular access site 110 and the target site 130.

A success factor representing a tortuosity of a portion of the vasculature between the vascular access site 110 and the target site 130, can be determined from received image data 140, such as CTA, MRA, and DSA image data, for example. Tortuous vessel segments may be detected in such image data using detection techniques such as computing overall curvature, computing curvature sign changes, or other techniques including machine learning techniques. Tortuous vessels may also be identified using a neural network. The neural network may be trained in an unsupervised manner. For example, a generative neural network such as a variational autoencoder "VAE" may be trained on a dataset including non-tortuous vessels. A VAE learns the distribution over the latent representation of the training data. Therefore, by training the VAE on non-tortuous vessels, the distribution learned by the VAE can provide a good representation of vasculature without tortuosity. At inference, when vasculature without tortuosity is inputted into the VAE, its latent representation will be an inlier in the learned distribution. However, if vasculature with higher tortuosity than represented in the training data, is inputted into the VAE, its latent representation will be an outlier in the learned distribution. Tortuous segments in the vasculature between the potential access sites and the target site can therefore be identified by detecting the outlier predictions of the VAE.

A tortuosity of a portion of the vasculature may be determined based on a count of the total number of tortuous segments and/or a magnitude of the tortuosity, between each potential vascular access site 110_{1...n} and the target site 130. The tortuosity may then be used to determine a success factor affecting the ease of navigating the interventional device from the vascular access site 110 to the target site 130 via the vasculature. In this example, the success factor may increase with decreasing tortuosity, and so the success factor may be inversely related to the tortuosity of the portion of the vasculature.

A success factor representing a difficulty of passing a stenosis in the vasculature between the vascular access site 110 and the target site 130, may also be calculated based on image data. Navigating an interventional device past a stenosis can be complex, and may increase procedure time in view of the need to avoid dislodging stenosis material. The positions of stenoses may be identified in image data such as IVUS, OCT, CTA and MRA by detecting narrowed regions of a lumen in the image data.

A difficulty of passing a stenosis in the vasculature may be determined based on a count of the total number of stenoses, and/or a total length of the stenosis/ stenoses, and/or a minimum diameter of the stenosis/ stenoses between the vascular access site 110 and the target site 130. This difficulty of passing the stenosis may be used to determine a success factor affecting the ease of navigating the interventional device from the vascular access site 110 to the target site 130 via the vasculature. In this example, the success factor may decrease as a count of the total number of stenoses increases, and so the success factor may be inversely related to the difficulty of passing a stenosis in the vasculature.

A success factor representing a difficulty of passing an implanted device in the vasculature between the vascular access site 110 and the target site 130, may also be calculated based on image data. Navigating an interventional device past an implanted device can also be complex and can therefore increase procedure time. The presence of an implanted device may be detected in image data such as IVUS, OCT, CTA and MRA image data by detecting characteristic patterns of the implanted device in the image data, such as stent struts. A success factor representing a difficulty of passing an implanted device, can be calculated in a similar manner as for a stenosis, and likewise used to calculate the success factor.

A success factor representing a difficulty of passing a calcification in the vasculature between the vascular access site 110 and the target site 130, may also be calculated based on image data. Vascular calcifications constrict vessels, hampering the navigation of interventional devices and increasing the risk of complications for a patient. The navigation of interventional devices past calcifications can cause the calcification to break off, resulting in downstream embolus and vascular ischemia. The positions of calcifications may be identified in image data such as IVUS, OCT, CTA, PET, and MRA image data. Calcifications can be identified in such image data using segmentation techniques, such as a U-Net, region growing, and thresholding, or using feature detection techniques. Neural networks may be trained to detect calcifications in a supervised manner using image data from clinical investigations on a variety of patients and in which the ground truth is annotated.

In the operation S140 the vascular access site 110 is identified based on the computed success metrics. This may involve comparing the computed success metrics 150 for each of the potential vascular access sites 110_{1...n} and identifying the vascular access site 110 having the highest success metric. The result of these operations is therefore to identify an optimal vascular access site for the target site. The optimal vascular access site may then be recommended to a physician. The optimal vascular access site is provided in a consistent and efficient manner, thereby obviating some of the existing challenges in determining an optimal access site.

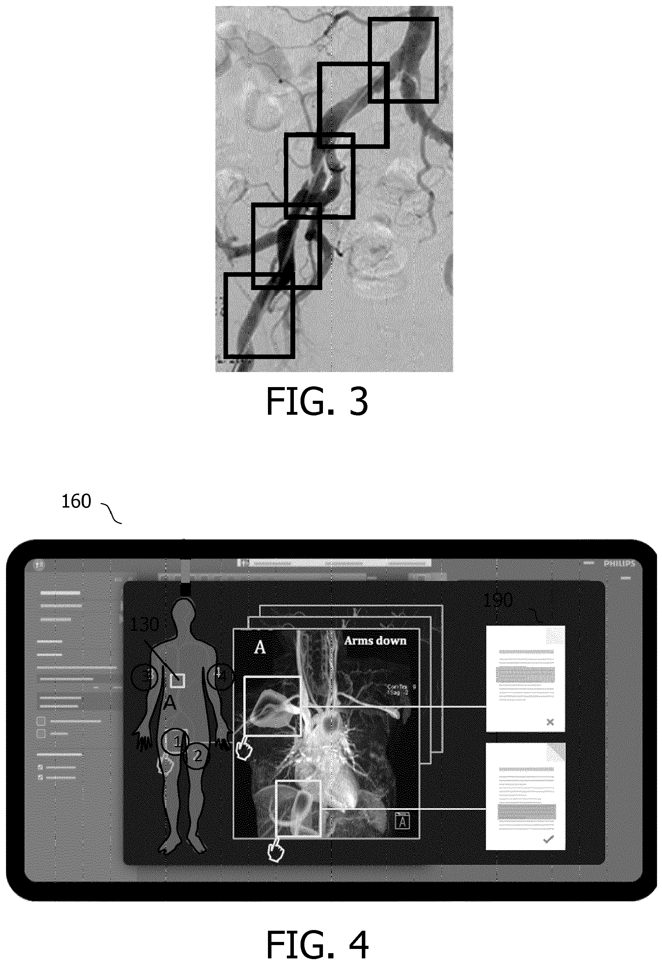

The optimal vascular access site 110 may be identified in the operation S140 in various ways. For example, the success metric for the optimal vascular access site 110, and/or the success metrics 150 for each of the potential vascular access sites 110_{1..n}, may be outputted to a display. The success metrics may be outputted to the display 230 illustrated in Fig. 2, or to another display, such as a display of a tablet, or of an augmented/virtual reality device, and so forth. The success metrics may be outputted via the graphical user interface illustrated in Fig. 4. The success metrics may be outputted numerically, or graphically. For example, the vascular access site with the highest success metric may be identified with a color in order to distinguish this vascular access site from other vascular access sites. The optimal vascular access site may also be identified in an anatomical image.

The operation of identifying S140 the vascular access site 110 may include providing a ranking of the potential vascular access sites 110_{1..n} based on the computed success metrics 150. Providing such a ranking permits a physician to choose between a highest-ranked access site and a lower-ranked access site. The ranking may be provided in the form of a table, or a numerical order indicating the hierarchy in the ranking. The ranking may alternatively be indicated graphically by displaying the potential vascular access sites 110_{1..n} on an anatomical image, and indicating their ranking on the anatomical image. In a related example, the operation of providing a ranking of the potential vascular access sites 110_{1..n}, includes:
outputting an anatomical image representing the target site 130 and the potential vascular access sites 110_{1..n}, and for each potential vascular access site, identifying in the anatomical image a location of at least a dominant feature affecting one or more of the success factors 150_{1..k}.

Such a dominant feature may for example be a tortuous portion of the vasculature. The dominant feature may for example be identified by means of an attention map, or a bounding box. An example of such a bounding box is illustrated in Fig. 3, which is a schematic diagram illustrating an example of an anatomical image including an identification of a location of a dominant feature affecting one or more of the success factors, in accordance with some aspects of the present disclosure. With reference to Fig. 3, portions of the vasculature with relatively higher levels of tortuosity are identified by the bounding box with dashed lines, whereas portions of the vasculature with relatively lower levels of tortuosity are identified in the bounding boxes with solid white lines. Outputting an anatomical image in this manner, may assist a user to understand why a particular vascular access site is considered to have a low success factor.

In a related example, the operation of identifying S140 the vascular access site 110 includes:
providing the ranking of the potential vascular access sites 110_{1..n} on a graphical user interface 160;
receiving user input indicative of the location of at least a dominant feature affecting one or more of the success factors 150_{1..k}; and
in response to the user input, outputting patient data 190 relating to the dominant feature to the graphical user interface 160.

This example is illustrated with reference to Fig. 4, which is a schematic diagram illustrating an example of a graphical user interface 160 for providing a ranking of potential vascular access sites, in accordance with some aspects of the present disclosure. With reference to Fig. 4, the graphical user interface, GUI, 160, includes an anatomical image toward the left-hand side of the drawing. In this example, the target site 130 is identified on the anatomical image by means of a square symbol, and the potential vascular access sites are identified by means of circles. The potential vascular access sites are also ranked as 1..4 based on their success metrics. The location of a dominant feature affecting one or more of the success factors 150_{1..k} is also indicated in Fig. 4 by means of the symbol "A". In this example, the user may use a user input device, such as a mouse or a touchscreen, to select the dominant feature represented by the symbol "A". Consequently, patient data 190 relating to the dominant feature is outputted to the GUI, as displayed in the GUI towards the right-hand side of Fig. 4. The patient data 190 that is outputted may for example include the image data from which the success metric was calculated for the access site. Alternatively, the outputted patient data may include electronic health record data such as a physician's report on a previous interventional procedure that has been performed at this location.

As mentioned above, the success metric for the potential vascular access sites 110_{1..n} may be based on one or more success factors. If the success metric is based on a single success factor, such as for example the tortuosity of the portion of the vasculature, the success metric may simply be equated with the success factor. The success metrics may then simply be compared for the vascular access sites, and the vascular access site 110 having the highest success metric may be outputted as the optimal, or recommended, vascular access site 110. However, if the success metrics for the potential vascular access sites 110_{1..n} are based on multiple different success factors 150_{1..k}, the individual success factors may be weighted to provide the success metric 150 for the potential vascular access site. The success metrics 150 may then be compared for the vascular access sites, and the vascular access site 110 having the highest success metric may be outputted as the optimal, or recommended, vascular access site 110.

In one example, the success factors may be weighted by setting the values of the weights of the individual success factors to fixed values. The values of the weights may be set such that the presence of calcifications and implanted devices result in a relatively lower success metric, whilst the absence of high tortuosity results in a relatively higher success metric, for example. In another example, the values of the weights may have variable values. For example, the values of the weights may be set by designing rules based on the values of each of the individual success factors 150_{1.k} such that the resulting success metric 150 matches an expert assessment of the ease of navigating the interventional device from the vascular access site to the target site via the vasculature. A neural network may be trained to generate the values of the weights based on the values of each of the individual success factors 150_{1.k}, or based on the image data from which the individual success factors 150_{1.k} are generated, such that the resulting success metric 150 matches an expert assessment of the ease of navigating the interventional device from the vascular access site to the target site via the vasculature. A supervised learning approach may be used to train the neural network to generate the resulting success metric 150 based on how experts consider the various individual success factors 150_{1.k} to influence the resulting success metric 150.

In some examples, the success metric 150 that is computed in the operation S130 also represents an outcome of performing a vascular intervention at the target site 130 using the vascular access site 110. In these examples, success factors that affect the outcome, such as for example the duration of a vascular intervention, the risk of complications such as bleeding and ischemic events, the likelihood of having to repeat the procedure, and other factors resulting from navigating the vasculature between the vascular access site 110 and the target site 130, may also be taken into account in computing the success metric 150. Values for each of these success factors may be obtained from clinical studies, such as reported in the document mentioned above by Jolly, S. et al., entitled "Radial versus femoral access for coronary angiography or intervention and the impact on major bleeding and ischemic events: A systematic review and meta-analysis of randomized trials," American Heart Journal 157(1): 132-140 (2009). These success factors may be included in the success metric 150 by weighting one or more of these factors with weights, the values of which are determined as described for the success factors 150_{1..k} above. By incorporating the outcome of performing a vascular intervention at the target site 130 using the vascular access site 110, into the success metric in this manner, a more evidence-based recommendation of the optimal vascular access site may be provided.

In some examples, a neural network is used to a determine the success metric 150 and/or to identify the optimal vascular access site 110. In one example, the operation of computing S130 a success metric 150 and/or the identifying S140 the vascular access site 110, is determined by inputting the target site 130, and the image data 140 representing the at least a portion of the vasculature, into at least one neural network 170. The at least one neural network 170 is trained to predict the success metric 150 and/or to identify the vascular access site 110, based on the inputted target site 130 and the image data 140.

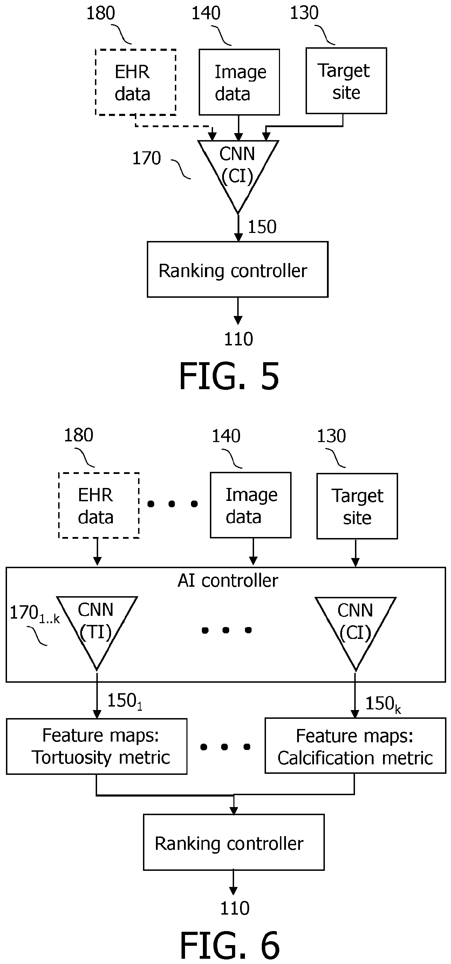

Fig. 5 is a schematic diagram illustrating a first example of a neural network 170 for use in identifying a vascular access site for inserting an interventional device in order to reach a target site 130 in a vasculature, in accordance with some aspects of the present disclosure. With reference to Fig. 5, in this example a single neural network 170 is used to predict the success metric 150 for each potential vascular access sites 110_{1..n}, and the success metric is calculated based on a single success factor "CI". The neural network 170 may include one or more architectures such as convolutional neural networks "CNNs", recurrent neural networks "RNNs", variational autoencoders "VAEs", transformers, and so forth. In this example, the success factor represents a difficulty of passing a calcification in the vasculature between the vascular access site 110 and the target site 130. In this example, image data 140 representing at least a portion of the vasculature, as well as an identification of the target site 130, are inputted into the neural network 170, and used to predict the success factors for each of multiple potential vascular access sites 110_{1..n}. As mentioned above, the image data 140 may include image data such as IVUS, OCT, CTA or MRA image data. The image data 140 may represent the vascular access site, the target site, or a portion of the vasculature between the vascular access site and the target site.

The predicted success factors 150 may then be ranked by the ranking controller illustrated in Fig. 5 in order to identify an optimal vascular access site 110. The ranking controller compares each of the individual success factors in order to identify the optimal vascular access site 110 and/or generate a ranking of the vascular access sites. The functionality of the ranking controller may be provided by one or more processors, such as the one or more processors 210 illustrated in Fig. 2.

In this example, a calcification was used to illustrate the type of features that the neural network may be trained to recognize. However, the single neural network may be trained to recognize multiple features that represent a difficulty of navigating through the vasculature between the vascular access site 110 and the target site 130, including calcifications, stenoses, tortuosity, and so forth. More generally, the advantage of using a single neural network to predict the success factors is that it implicitly learns the relevant features associated with the success metric 150. The neural network may therefore learn features associated with the success factor that were not anticipated at the training stage, and therefore not accounted for by training the neural network based on a single type of data, such as calcification data.

Fig. 6 is a schematic diagram illustrating a second example of a neural network 170 for use in identifying a vascular access site for inserting an interventional device in order to reach a target site 130 in a vasculature, in accordance with some aspects of the present disclosure. With reference to Fig. 6, in this example, multiple success factors 150_{1..k} are predicted by corresponding neural networks 170_{1..k} for each potential vascular access site 110_{1..n}. In this example, the multiple success factors, i.e. k>1, may represent features such as: a tortuosity of a portion of the vasculature between the vascular access site 110 and the target site 130, i.e. "TI", a difficulty of passing a stenosis in the vasculature between the vascular access site 110 and the target site 130, a difficulty of passing an implanted device in the vasculature between the vascular access site 110 and the target site 130, and a difficulty of passing a calcification in the vasculature between the vascular access site 110 and the target site 130, i.e. "CI". As illustrated in Fig. 6, an AI controller may be used to control the operation of the various neural networks 170_{1..k} . The AI controller may control the inputting of data into the neural networks for each of the potential vascular access sites. The functionality of the AI controller may be provided by one or more processors, such as the one or more processors 210 illustrated in Fig. 2.

In the example illustrated in Fig. 6, image data 140 representing at least a portion of the vasculature, and an identification of the target site 130, are inputted into the AI controller, and subsequently into the relevant neural network 170_{1..k} and used to predict multiple success factors 150_{1..k} for each of multiple potential vascular access sites 110_{1..n}. The predicted success factors 150_{1..k} for the different features are then weighted by the ranking controller to provide a success metric for each potential vascular access site 110_{1..n}. The ranking controller then compares the success metrics for the potential vascular access site 110_{1..n} in order to identify the optimal vascular access site 110 and/or generate a ranking of the vascular access sites.

Thus, in the example illustrated in Fig. 6, the success metrics 150, are determined by inputting the target site 130, and the image data 140 representing the at least a portion of the vasculature, into a plurality of neural networks 170_{1..k}; and a separate neural network is trained to predict each of the success factors 150_{1..k} based on the inputted target site 130, and the image data 140. Training a separate neural network to predict each success factor separately in this manner may simplify their training.

The neural networks described above with reference to Fig. 5 and Fig. 6 may also output their respective confidence values. The confidence values represent a confidence of the predictions made by the neural network(s) 170, thereby permitting decisions to be made based on the predicted success factors. The confidence of the neural network may be low for a vascular access site if there is insufficient image data for that site. By way of an example, if the confidence is low for the optimal vascular access site, it might be decided not to rely upon this access site, and to instead use an alternative access site that has a higher confidence value.

An example of a technique for generating confidence values associated with a neural network's predictions is disclosed in a document by Ramalho, T. et al., entitled "Density estimation in representation space to predict model uncertainty", https://arxiv.org/pdf/1908.07235.pdf. The neural network may be trained in accordance with this technique to generate confidence values such that when the neural network is presented with an image that is very different from its training dataset, the neural network it is able to recognize this and the neural network outputs a low confidence value. The technique described in this document generates confidence values by estimating the training data density in representation space, and determining whether the trained network is expected to make a correct prediction for the input by measuring the distance in representation space between the input and its closest neighbors in the training set. Alternative techniques may also be used to generate confidence values associated with the predictions of the neural network. The dropout technique may be used, for example. The dropout technique involves iteratively inputting the same data into a neural network and determining the neural network's output whilst randomly excluding a proportion of the neurons from the neural network in each iteration. The outputs of the neural network are then analyzed to provide mean and variance values. The mean value represents the final output, and the magnitude of the variance indicates whether the neural network is consistent in its predictions, in which case the variance is small, or whether the neural network was inconsistent in its predictions, in which case the variance is larger.

The neural network(s) described above with reference to Fig. 5 and Fig. 6 may also predict the success metrics based on additional data, including electronic health record, EHR data. This is indicated in each of Fig. 5 and Fig. 6 by way of item 180 labelled "EHR data" in dashed outline. In one example, the at least one neural network 170 is trained to predict the success metric 150 and/or to identify the vascular access site 110, based further on electronic health record data 180; and the method described with reference to Fig. 1 includes:
receiving electronic health record data 180 relating to the vasculature; and
inputting the electronic health record data into the at least one neural network 170.

EHR data may include information relating to body mass index, smoking history, age, gender, and so forth. EHR data may also include information from clinical investigations on a patient, such as vessel characteristics, tortuosity, thickness, artifacts within vessels such as calcifications, the existence of implanted devices such as stents, the outcomes of historic procedures on the patient, such as bleeding incidence, and so forth. EHR data may include information that can help inform physicians about potential difficulties they might encounter during endovascular navigation. For example, smoking has been shown to constrict vessels and to be highly correlated with atherosclerotic changes in vessels, i.e. a build-up of plaque, as described in a document by Ambrose, J., et al., entitled "The pathophysiology of cigarette smoking and cardiovascular disease: an update", J. Am. Coll. Cardiol. 43(10): 1731-1737 (2004). doi: 10.1016/j.jacc.2003.12.047. Therefore, features such as vessel width and vessel tortuosity may present different levels of severity based on patients' smoking history. Similarly, EHR data may include information from historic procedures that can better inform the choice of access site. For example, historic procedure reports may indicate instances of bleeding or details about implanted devices.

The EHR data may be stored on a database, such as a picture archiving and communication system "PACS" system. In this example, the EHR data may therefore be received from a database. The EHR data may be received by a processor, such as the one or more processors 210 illustrated in Fig. 2. The electronic health record data 180 may be processed using a natural language processing "NLP" technique prior to inputting the electronic health record data into the at least one neural network 170. NLP is a branch of artificial intelligence that allows computers to understand text in much the same way as human beings. NLP techniques may be used to convert text or image data into computer-readable data, and to perform tasks such as word disambiguation in order to comprehend the text. NLP may be used to identify keywords that can better inform physicians on the choice of access site. NLP may also be used to learn contextual relationships between words and accompanying images. The use of NLP may advantageously reduce the workload for physicians to manually label or review such data, and also reduces the chance of potentially critical patient information being overlooked.

In one example, the image data that is inputted into the neural network(s) 170 includes ultrasound image data that is generated prior to inserting the interventional device (120) into the identified vascular access site. Ultrasound image data is increasingly used immediately prior to the start of interventional procedures in order to visualize the vascular structure around an access site. As an example, for femoral access, an ultrasound probe may be used to find the common femoral artery. In this example, the additional ultrasound imaging information is also processed by the neural network(s) in order to re-evaluate the optimal vascular access site that is identified. For example, if, in assessing the difficulty of passing a calcification in the vasculature between the vascular access site 110 and the target site 130, i.e. "CI", calcification or plaque is identified near this access site, this can lower the success metric 150 at the current potential access site 110_{1..n}, which may result in a different vascular access site being optimal. Alternatively, by observing changes in the success metric 150 as the ultrasound probe is moved around near the optimal access site and choosing the exact location with the highest success metric 150, a precise location for incision at the access site can be identified.

In a similar manner, in one example, image data that is generated during an interventional procedure, such as for example ultrasound, or X-ray image data, may be inputted into the one or more neural networks 170, and used to periodically update the predicted success factors for the potential vascular access sites. In so doing, additional information from this image data may be used to confirm that an interventional procedure is being carried out using the optimal vascular access site on the basis of the most up-to date information, or to otherwise recommend an alternative vascular access site. In this example, the suitability of an initially selected access point may be constantly reassessed whilst a user navigates the interventional device through the vasculature.

In one example, the neural network(s) are also trained to predict a simulated path for the interventional device. The simulated path is then used to calculate a complexity metric for navigating the interventional device. The complexity metric is then used to calculate the success metric. In this example, the at least one neural network 170 is further trained to predict a simulated path for the interventional device to reach the target site 130 in the vasculature from each of the potential vascular access sites 110_{1..n}; and the at least one neural network 170 is trained to compute the success metric 150 and/or to identify the vascular access site 110, based further on a complexity metric representing a difficulty of reaching the target site 130 in the vasculature from each of the potential vascular access sites 110_{1..n} with the interventional device.

Using the complexity metric in the calculation of the success metric takes account of the capabilities of navigating a particular interventional device along the paths between the potential vascular access sites, and the target site. The complexity metric may be based on factors that positively affect the success metric, such as taking a step toward the target site, and factors that negatively affect the success metric, such as a count of the number of times the interventional device touches the lumen walls, the total number of steps taken to reach the target site, and so forth. If the interventional device frequently touches the lumen walls, this indicates narrow or tortuous lumen. If a high number of steps are used to reach the target site, this might also indicate tortuous lumens and/or a larger path length between the vascular access site and target site.

The training of the neural network(s) 170 is now detailed below.

In general, the training of a neural network involves inputting a training dataset into the neural network, and iteratively adjusting the neural network's parameters until the trained neural network provides an accurate output. Training is often performed using a Graphics Processing Unit "GPU" or a dedicated neural processor such as a Neural Processing Unit "NPU" or a Tensor Processing Unit "TPU". Training often employs a centralized approach wherein cloud-based or mainframe-based neural processors are used to train a neural network. Following its training with the training dataset, the trained neural network may be deployed to a device for analyzing new input data during inference. The processing requirements during inference are significantly less than those required during training, allowing the neural network to be deployed to a variety of systems such as laptop computers, tablets, mobile phones and so forth. Inference may for example be performed by a Central Processing Unit "CPU", a GPU, an NPU, a TPU, on a server, or in the cloud.

The process of training the neural network(s) 170 described above therefore includes adjusting its parameters. The parameters, or more particularly the weights and biases, control the operation of activation functions in the neural network. In supervised learning, the training process automatically adjusts the weights and the biases, such that when presented with the input data, the neural network accurately provides the corresponding expected output data. In order to do this, the value of the loss functions, or errors, are computed based on a difference between predicted output data and the expected output data. The value of the loss function may be computed using functions such as the negative log-likelihood loss, the mean squared error, or the Huber loss, or the cross entropy loss. During training, the value of the loss function is typically minimized, and training is terminated when the value of the loss function satisfies a stopping criterion. Sometimes, training is terminated when the value of the loss function satisfies one or more of multiple criteria.

Various methods are known for solving the loss minimization problem such as gradient descent, Quasi-Newton methods, and so forth. Various algorithms have been developed to implement these methods and their variants including but not limited to Stochastic Gradient Descent "SGD", batch gradient descent, mini-batch gradient descent, Gauss-Newton, Levenberg Marquardt, Momentum, Adam, Nadam, Adagrad, Adadelta, RMSProp, and Adamax "optimizers" These algorithms compute the derivative of the loss function with respect to the model parameters using the chain rule. This process is called backpropagation since derivatives are computed starting at the last layer or output layer, moving toward the first layer or input layer. These derivatives inform the algorithm how the model parameters must be adjusted in order to minimize the error function. That is, adjustments to model parameters are made starting from the output layer and working backwards in the network until the input layer is reached. In a first training iteration, the initial weights and biases are often randomized. The neural network then predicts the output data, which is likewise, random. Backpropagation is then used to adjust the weights and the biases. The training process is performed iteratively by making adjustments to the weights and biases in each iteration. Training is terminated when the error, or difference between the predicted output data and the expected output data, is within an acceptable range for the training data, or for some validation data. Subsequently the neural network may be deployed, and the trained neural network makes predictions on new input data using the trained values of its parameters. If the training process was successful, the trained neural network accurately predicts the expected output data from the new input data.

In one example, the at least one neural network 170 is trained to predict the success metric 150 and/or to identify the vascular access site 110, by:
receiving S210 training data, the training data comprising image data 140' representing a portion of a vasculature and a corresponding target site 130', for each of a plurality of subjects;
receiving S220 ground truth data, the ground truth data representing an ease of navigating the interventional device from the vascular access site 110 to the target site 130' via the portion of the vasculature, for each of the target sites in the training data; and
for a plurality of the subjects:
   inputting S230 the training data and the ground truth data; and adjusting S240 parameters of the neural network until a value of a loss function representing a difference between a success metric 150 predicted by the neural network 170 and/or a vascular access site 110 predicted by the neural network 170, and the ground truth data, meets a stopping criterion.

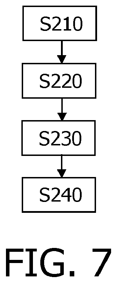

This training method is illustrated in Fig. 7, which is a flowchart illustrating an example of a method of training a neural network to predict a success metric and/or to identify a vascular access site, in accordance with some aspects of the present disclosure. An example is provided for training the neural network(s) to predict a single success factor based on a difficulty of passing a calcification in the vasculature between the vascular access site 110 and the target site 130. In this example, the training data may for example include IVUS image data for portions of the vasculature between a vascular access site, and a target site, for multiple patients. Calcifications are often annotated in IVUS image data. The data on the calcifications may be presented to a physician who assesses the data and provides a ground truth success factor for the vascular access site. The ground truth success factor may be based on features such as the locations, total length, type, and minimum diameter, of the calcifications. The ground truth success factor represents an ease of navigating the interventional device from the vascular access site 110 to the target site 130'. The neural network 170 is then trained in accordance with the backpropagation method described above to predict the success factors for the training data for the multiple patients.

Similarly, when multiple success factors are taken into account, a single neural network may be trained to predict a physician-assessed overall success metric that takes into account the multiple factors. The single neural network may also be trained in an unsupervised manner using a generative neural network like a variational autoencoder "VAE" with images of portions of vasculature containing no, or negligible, features associated with a difficulty of navigating an interventional device from the vascular access site 110 to the target site 130', as assessed by an expert physician or physicians. Therefore, the VAE learns the distribution over the latent representation of the training data containing vasculature without tortuosity or calcifications or stenoses and so on. When vasculature without tortuosity or calcifications or stenoses and so forth are inputted into the trained VAE, the latent representations of these inputs will be inliers in the learned distribution. However, if vasculature with tortuosity or calcifications or stenoses and so on are inputted into the trained VAE, their latent representations will be outliers in the learned distribution. Further, since VAEs are trained to reconstruct the inputted data, the trained VAE will include reconstruction errors in regions of images containing features that were not present in the training data, allowing the identification of such regions of high reconstruction error. The more tortuous the vasculature (or, similarly, the larger the calcification or stenosis), the larger the reconstruction error. Quantification of these errors and/or number of regions including errors will allow a single neural network to predict overall success metrics that take multiple factors into account. Additionally, this allows the network to learn features associated with the success factor that were not anticipated.

When multiple neural networks are trained to predict the success metric, each individual neural network may be trained individually with the physician-assessed ground truth success factors for a particular feature, such as:
a tortuosity of a portion of the vasculature between the vascular access site 110 and the target site 130;
a difficulty of passing a stenosis in the vasculature between the vascular access site 110 and the target site 130;
a difficulty of passing an implanted device in the vasculature between the vascular access site 110 and the target site 130;
a difficulty of passing a calcification in the vasculature between the vascular access site 110 and the target site 130.

In some examples, the ground truth data may include annotations of image features in the form of binary masks or bounding boxes representing a difficulty of navigating an interventional device from the vascular access site 110 to the target site 130' such as calcifications or stenoses and so on. In this example, the neural network 170 or networks 170_{1..k} may be trained to predict these annotations; and the adjusting parameters of the neural network 170 or networks 170_{1..k}, is repeated until a value of a loss function representing a difference between an annotation predicted by the neural network 170 or networks 170_{1..k}, and the ground truth annotation, meets a stopping criterion. In some examples, the ground truth data may include a ranking of multiple potential vascular access sites. For instance, identified factors that represent a difficulty of navigating an interventional device from the vascular access site 110 to the target site 130' such as tortuosity or calcifications and so on may be presented to experts who can rank various access sites based on the influence of the identified features on navigation and/or procedure success. This ranking may also be used to train the neural network to predict the ranking of the vascular access sites. Thus, in one example, the ground truth data comprises a ranking of vascular access sites 110 for each of the target sites 130. In this example, the neural network 170 is trained to predict a ranking of the vascular access sites for each inputted target site 130'; and the adjusting parameters of the neural network 170, is repeated until a value of a loss function representing a difference between a ranking of the vascular access sites predicted by the neural network, and the ground truth ranking of the access sites, meets a stopping criterion. In some examples, the ground truth includes expert labels determining which images contain features representing a difficulty of navigating an interventional device from the vascular access site 110 to the target site 130' such as tortuosity or calcifications and so on and which images do not contain such features. In this example, the neural network 170 or networks 170_{1..k} may be trained in an unsupervised manner with images that do not contain features representing a difficulty of navigating an interventional device from the vascular access site 110 to the target site 130'. For instance, a VAE may be trained to reconstruct the inputted training data by adjusting parameters of the neural network 170 or networks 170_{1..k} until a value of a loss function representing a difference between the reconstructed image and the inputted image meets a stopping criterion, and a value of a loss function representing a difference between the predicted components of a distribution over the latent representation of the inputted training data and the components of a standard distribution meets a stopping criterion. When images containing features representing a difficulty of navigating an interventional device from the vascular access site 110 to the target site 130' are inputted into such a trained neural network, they are identified as outliers in the learned distribution over the latent representation of the training data.

In one example, a computer program product is provided. The computer program product includes instructions which when executed by one or more processors, cause the one or more processors to carry out a method of identifying a vascular access site 110 for inserting an interventional device 120 in order to reach a target site 130 in a vasculature. The method includes:
receiving S110 input indicative of the target site 130;
receiving S120 image data 140 representing at least a portion of the vasculature; and
for a plurality of potential vascular access sites 110_{1..n}:
   computing S130, based on the image data 140, a success metric 150 representing an ease of navigating the interventional device 120 from the vascular access site 110 to the target site 130 via the vasculature; and
   identifying S140 the vascular access site 110 based on the computed success metrics.

As mentioned above, features of the computer implemented method, may also be implemented in a system. Thus, in one example, a system 200 for identifying a vascular access site 110 for inserting an interventional device 120 in order to reach a target site 130 in a vasculature, is provided. The system includes one or more processors 210 configured to:
receive S110 input indicative of the target site 130;
receive S120 image data 140 representing at least a portion of the vasculature; and
for a plurality of potential vascular access sites 110_{1..n}:
   compute S130, based on the image data 140, a success metric 150 representing an ease of navigating the interventional device 120 from the vascular access site 110 to the target site 130 via the vasculature; and
   identify S140 the vascular access site 110 based on the computed success metrics.

An example of this system 200 is illustrated in Fig. 2. The system includes one or more processors 210. The one or more processors may also perform one or more additional operations described above with reference to the flowchart in Fig. 1. The system 200 may also include one or more of: a projection X-ray imaging system 220 for providing the image data 140; a display 230 for displaying the computed success metric 150, the identified optimal vascular access site 110, the graphical user interface 160, and so forth; an interventional device 120 for insertion into the vasculature; and a user input device configured to receive user input (not illustrated in Fig. 2), such as a keyboard, a mouse, a touchscreen, a joystick, and so forth. The system 200 may also include an endovascular robot configured to navigate an interventional device from the optimal access site to the target site.

The above examples are to be understood as illustrative of the present disclosure, and not restrictive. Further examples are also contemplated. For instance, the examples described in relation to computer-implemented methods, may also be provided by the computer program product, or by the computer-readable storage medium, or by the system 200, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A computer-implemented method of identifying a vascular access site (110) for inserting an interventional device (120) in order to reach a target site (130) in a vasculature, the method comprising:
receiving (S110) input indicative of the target site (130);
receiving (S120) image data (140) representing at least a portion of the vasculature; and
for a plurality of potential vascular access sites (110_{1..n}):
computing (S130), based on the image data (140), a success metric (150) representing an ease of navigating the interventional device (120) from the vascular access site (110) to the target site (130) via the vasculature; and
identifying (S140) the vascular access site (110) based on the computed success metrics.

2. The computer-implemented method according to claim 1, wherein the success metric (150) further represents an outcome of performing a vascular intervention at the target site (130) using the vascular access site (110).

3. The computer-implemented method according to claim 1 or claim 2, wherein the computing (S130) a success metric (150), comprises:
computing values of a plurality of success factors (150_{1..k}) affecting i) the ease of navigating the interventional device from the vascular access site (110) to the target site (130) via the vasculature and/or ii) the outcome of performing the vascular intervention at the target site (110) using the vascular access site (110); and
weighting the computed values to provide the success metric (150).

4. The computer-implemented method according to claim 3, wherein the success factors (150_{1..k}) represent one or more of the following features:
a tortuosity of a portion of the vasculature between the vascular access site (110) and the target site (130);
a difficulty of passing a stenosis in the vasculature between the vascular access site (110) and the target site (130);
a difficulty of passing an implanted device in the vasculature between the vascular access site (110) and the target site (130);
a difficulty of passing a calcification in the vasculature between the vascular access site (110) and the target site (130).

5. The computer-implemented method according to any one of claims 1-4
wherein the identifying (S140) the vascular access site (110), comprises:
providing a ranking of the potential vascular access sites (110_{1..n}); and
wherein the ranking is based on the computed success metrics (150).

6. The computer-implemented method according to claim 5 when dependent on claim 4, wherein the providing a ranking of the potential vascular access sites (110_{1..n}), comprises:
outputting an anatomical image representing the target site (130) and the potential vascular access sites (110_{1..n}), and for each potential vascular access site, identifying in the anatomical image a location of at least a dominant feature affecting one or more of the success factors (150_{1..k}).

7. The computer-implemented method according to claim 6, wherein the identifying (S140) the vascular access site (110), comprises:
providing the ranking of the potential vascular access sites (110_{1..n}) on a graphical user interface (160);
receiving user input indicative of the location of at least a dominant feature affecting one or more of the success factors (150_{1..k}); and
in response to the user input, outputting patient data (190) relating to the dominant feature to the graphical user interface (160).

8. The computer-implemented method according to any previous claim, wherein the computing (S130) a success metric (150) and/or the identifying (S140) the vascular access site (110), is determined by inputting the target site (130), and the image data (140) representing the at least a portion of the vasculature, into at least one neural network (170); and
wherein the at least one neural network (170) is trained to predict the success metric (150) and/or to identify the vascular access site (110), based on the inputted target site (130) and the image data (140).

9. The computer-implemented method according to claim 8, wherein the at least one neural network (170) is trained to predict the success metric (150) and/or to identify the vascular access site (110), based further on electronic health record data (180); and wherein the method further comprises:
receiving electronic health record data (180) relating to the vasculature; and
inputting the electronic health record data into the at least one neural network (170).

10. The computer-implemented method according to claim 9, wherein the electronic health record data (180) is processed using a natural language processing technique prior to inputting the electronic health record data into the at least one neural network (170).

11. The computer-implemented method according to claim 8 when dependent on claim 3, wherein the computing (S130) a success metric (150), is determined by inputting the target site (130), and the image data (140) representing the at least a portion of the vasculature, into a plurality of neural networks (170_{1..k}); and
wherein a separate neural network is trained to predict each of the success factors (150_{1..k}) based on the inputted target site (130), and the image data (140).

12. The computer-implemented method according to any one of claims 8 - 11, wherein the image data (140) comprises ultrasound image data generated prior to inserting the interventional device (120) into the identified vascular access site.

13. The computer-implemented method according to any one of claims 8 - 11, wherein the at least one neural network (170) is further trained to predict a simulated path for the interventional device to reach the target site (130) in the vasculature from each of the potential vascular access sites (110_{1..n}); and
wherein the at least one neural network (170) is trained to compute the success metric (150) and/or to identify the vascular access site (110), based further on a complexity metric representing a difficulty of reaching the target site (130) in the vasculature from each of the potential vascular access sites (110_{1..n}) with the interventional device.

14. The computer-implemented method according to any one of claims 8 - 13, wherein the at least one neural network (170) is trained to predict the success metric (150) and/or to identify the vascular access site (110), by:
receiving (S210) training data, the training data comprising image data (140') representing a portion of a vasculature and a corresponding target site (130'), for each of a plurality of subjects;
receiving (S220) ground truth data, the ground truth data representing an ease of navigating the interventional device from the vascular access site (110) to the target site (130') via the portion of the vasculature, for each of the target sites in the training data; and
for a plurality of the subjects:
inputting (S230) the training data and the ground truth data; and adjusting (S240) parameters of the neural network until a value of a loss function representing a difference between a success metric (150) predicted by the neural network (170) and/or a vascular access site (110) predicted by the neural network (170), and the ground truth data, meets a stopping criterion.

15. The computer-implemented method according to claim 14, wherein the ground truth data comprises a ranking of vascular access sites (110) for each of the target sites (130); and
wherein the neural network (170) is trained to predict a ranking of the vascular access sites for each inputted target site (130'); and
wherein the adjusting parameters of the neural network (170), is repeated until a value of a loss function representing a difference between a ranking of the vascular access sites predicted by the neural network, and the ground truth ranking of the access sites, meets a stopping criterion.
